# EUROPÄISCHE PATENTSCHRIFT

(11) **EP 0 452 442 B1**
(45) Veröffentlichungstag und Bekanntmachung des Hinweises auf die Patenterteilung: **28.02.1996**
(21) Anmeldenummer: 90915441.1
(22) Anmeldetag: 02.11.1990
(51) Int. Cl.: A61B 17/58, A61B 17/86

(54) **KNOCHENSCHRAUBE**
BONE SCREW
VIS A OS

(30) Priorität: 03.11.1989 DE 3936703
(43) Veröffentlichungstag der Anmeldung: 23.10.1991
(73) Patentinhaber: Biedermann, Lutz, D-78048 Villingen-Schwenningen (DE); Harms, Jürgen, Prof. Dr., D-76337 Waldbronn (DE)
(72) Erfinder: Biedermann, Lutz, D-78048 Villingen-Schwenningen (DE); Harms, Jürgen, Prof. Dr., D-76337 Waldbronn (DE)
(74) Vertreter: Prüfer, Lutz H., Dipl.-Phys.
(86) Internationale Anmeldenummer: EP9001835
(87) Internationale Veröffentlichungsnummer: WO9106252

(56) Entgegenhaltungen:
- EP-A- 0 226 701
- CH-A- 603 143
- DE-A- 2 063 650
- DE-A- 3 601 865
- DE-C- 3 614 101

## Beschreibung

Die Erfindung betrifft eine Knochenschraube mit einem Gewindeschaftteil und einem Kopf, wie sie insbesondere als Pedikelschraube Anwendung findet.

Eine solche Schraube ist beispielsweise aus der DE-36 14 101 C bekannt.

In bestimmten Fällen ist das Knochenmaterial, in welches die Knochenschraube einzusetzen ist, so weich, daß es schwierig ist, die Schraube ausreichend zu fixieren. Aus DE-A-2 063 650 ist bekannt, ein Verbindungselement durch Aufspreizen Seines distalen Endes im Knochen zu Verankern.

Aufgabe der Erfindung ist es, eine Knochenschraube mit einem Gewindeschaftteil und einem Kopf zu schaffen, mit der die Fixierung in einem Knochen gegenüber herkömmlichen derartigen Schrauben verbessert werden kann. Das Fixieren soll fein einstellbar sein.

Diese Aufgabe wird durch eine Knochenschraube der im Patentanspruch 1 gekennzeichneten Art gelöst.

Weiterbildungen der Erfindung ergeben sich aus den Unteransprüchen.

Weitere Merkmale und Zweckmäßigkeiten der Erfindung von Ausführungsbeispielen anhand der Figuren.

Von den Figuren zeigen:
- Fig. 1: eine Explosionszeichnung der Knochenschraube, teilweise in geschnittener Darstellung, und
- Fig. 2: die zusammengesetzte Knochenschraube mit einem Aufnahmeteil.

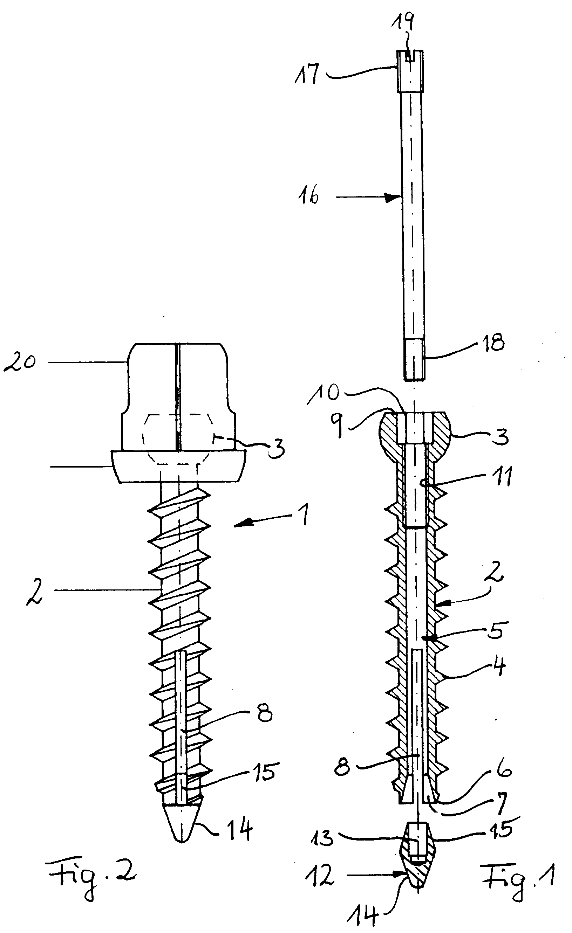

Die Knochenschraube 1 weist einen Gewindeschaftteil 2 und einen damit einstückig ausgebildeten Kopf 3 auf. Der Gewindeschaftteil weist ein üblicherweise für Knochenschrauben verwendetes Außengewinde 4 auf. Wie aus Fig. 1 ersichtlich ist, erstreckt sich durch das Innere der Schraube eine koaxial ausgerichtete Längsbohrung 5. Diese weist an ihrem dem Kopf 3 abgewandten freien Ende 6 eine sich zum freien Ende hin erweiternde koaxial angeordnete konische Ausnehmung 7 auf. In der Wandung des Gewindeschaftteiles sind vom freien Ende 6 ausgehend sich parallel zur Längsachse des Gewindeschaftteiles erstreckende Schlitze 8 vorgesehen. Diese erstrecken sich über eine Länge, in die einem mehrfach der sich in axialer Richtung ergebenden Länge der konischen Ausnehmung und wenigstens gleich dem zweifachen der Länge der konischen Ausnehmung ist. Vorzugsweise ist ein Paar solcher Schlitze vorgesehen, die um 180 Grad gegeneinander versetzt angeordnet sind.

Der Kopf weist auf seiner dem freien Ende gegenüberliegenden Seite eine sich im wesentlichen senkrecht zur Symetrieachse erstreckende ebene Fläche 9 auf, in die in axialer Richtung gesehen eine Ausnehmung 10 vorgesehen ist, die so ausgebildet ist, daß die Schraube mittels dieser Öffnung und eines damit zusammenwirkenen Schraubendrehers in einen Knochen eindrehbar ist. Diese Ausnehmung weist einen Durchmesser auf, der größer ist als der Durchmesser der Längsbohrung 5. An die Ausnehmung 10 schließt sich in der Längsbohrung 5 ein Abschnitt 11 mit einem Innengewinde an. Die Länge dieses Abschnittes ist vorzugsweise wenigstens gleich dem zweifachen der Länge der konischen Ausnehmung 7.

Wie aus Fig. 1 ersichtlich ist, ist die zu dieser Knochenschraube gehörende Spitze als getrenntes Spitzenteil 12 ausgebildet. Das Spitzenteil weist auf seiner dem Kopf zugewandten Seite eine koaxiale Bohrung 13 auf. Seine dem Kopf abgewandte Seite weist die der Spitze der Knochenschraube entsprechende Spitze 14 auf. Auf der der Spitze abgewandten Seite ist die Außenwandung mit einem konischen Abschnitt 15 versehen, der zu der konischen Ausnehmung 7 hin verjüngt verlauft und in seinen Abmessungen so ausgebildet ist, daß er in die konische Ausnehmung 7 hineinpaßt.

Ferner ist ein Schaft 16 vorgesehen, der an seinem einen Ende einen ersten Gewindeabschnitt 17 aufweist. Dieses Gewinde entspricht in Durchmesser und Steigung dem Innengewinde des Abschnittes 11, so daß es mit diesem zusammenwirken kann. Der übrige Teil des Schaftes 16 ist in seinem Durchmesser so ausgebildet, daß er gerade durch die Längsbohrung hindurchführbar ist. An seinem dem ersten Gewindeabschnitt 17 gegenüberliegenden Ende weist der Schaft 16 einen zweiten Gewindeabschnitt 18 auf. Die Bohrung 13 weist ein Innengewinde auf, welches in seinen Maßen dem Außengewinde des zweiten Gewindeabschnittes 18 entspricht. Der Gewindeabschnitt 18 mit dem zugehörigen Gewinde in der Bohrung 13 ist bezüglich seiner Steigrichtung entgegegengesetzt ausgebildet zu dem Gewinde des ersten Gewindeabschnittes 17 und dem zugehörigen Innengewinde im Abschnitt 11.

Der Schaft 16 ist so lang ausgebildet, daß er mit seinem zweiten Gewindeabschnitt 18 so weit in die konische Ausnehmung 7 hineinragt, daß das Spreizteil 12 so auf den Gewindeabschnitt aufschraubbar ist, daß die Spitze in der in Fig. 2 gezeigten Weise der normalen Spitze der Knochenschraube 1 entspricht, wenn der erste Gewindeabschnitt 17 bis zum Grund oder wenigstens nahe zum Grund des Gewindes des Abschnittes 11 eingeschraubt ist. Der Schaft weist an seinem am ersten Gewindeabschnitt angrenzenden Kopf eine Ausnehmung 19 zum Eingreifen mit einem Schraubendreher auf.

Im Betrieb wird zunächst der Schaft in die Längsbohrung 5 bis zum Grund des Abschnittes 11 eingeschraubt. Anschließend wird das Spreizteil 12 auf das in die konische Ausnehmung 7 hervorstehende Ende mit dem zweiten Gewindeabschnitt 18 aufgeschraubt, so daß die Schraube die übliche Spitze 14 in der aus Fig. 2 ersichtlichen Weise aufweist. Die Schraube kann dann gewünschtenfalls mit einem mit dem Kopf allseits beweglich verbundenen Aufnahmeteil 20 verbunden werden, wie es in der DE 37 11 013 C beschrieben ist und welches dazu dient, die Schraube mit Stangen zur Fixierung von Knochenteilen und insbesondere Wirbelsäulensegmenten Zu verbinden.

Ist die Knochenschraube in einen Knochen eingeschraubt, dann wird vom Kopfende her durch Eingriff mit der Ausnehmung 19 der Schaft 16 aus seiner Grundstellung zum Kopf 3 hin herausgeschraubt. Aufgrund der unterschiedlichen Steigungsrichtungen der beiden Gewindeabschnitte 17 und 18 und der zugehörigen Innengewinde bleibt die Verbindung zwischen zweitem Gewindeabschnitt 18 und Spreizteil fest, und das Spreizteil wird in den geschlitzten Bereich eingezogen und bewirkt, daß das freie Ende 6 des Gewindeschaftteiles 2 sich aufspreizt. Da der Bereich, in dem die Kraftübertragung vom Spreizteil auf den Gewindeschaftteil erfolgt, formstabil bleibt, also nicht selbst aufgespreizt wird, ist eine sehr genaue Verstellung möglich.

Bei einer gegenüber der oben beschriebenen Form abgewandelten Ausführungsform weist der Abschnitt 11 einen gegenüber den übrigen Bereich der Längsbohrung 5 größeren Durchmesser auf, wobei der erste Gewindeabschnitt 17 im Durchmesser entsprechend angepaßt ist.

Bei einer weiteren abgewandelten Ausführungsform weist der Abschnitt 11 einen kleineren Durchmesser auf als der sich bis zum freien Ende 6 hin anschließende Abschnitt der Längsbohrung 5. Der Durchmesser des ersten Gewindeabschnittes entspricht in seinen Abmessungen dem Innengewinde des Abschnittes 11. Der Schaft 16 und das Spreizteil 12 sind einstückig miteinander ausgebildet, und der Schaft 16 mit dem Spreizteil wird vom freien Ende 6 her in den Gewindeschaftteil 2 eingeschraubt. Der erste Gewindeabschnitt 17 ist in diesem Fall so lang ausgebildet, daß der Schaft aus einer oben beschriebenen Grundstellung, in der die Spitze 14 die in Fig. 2 gezeigte Normalstellung einnimmt, soweit zum Kopf hin verdrehbar ist, daß die gewünschte Aufspreizung durch das Einziehen des Spreizteiles in den geschlitzten Abschnitt erfolgt.

## Patentansprüche

1. Knochenschraube (1) mit einem Gewindeschaftteil (2)
und einem Kopf (3)
gekennzeichnet durch eine sich entlang der Längsachse des Gewindeschaftteiles (2) erstreckende Längsbohrung (5)
einen sich zu dem dem Kopf abgewandten freien Ende (6) erweiternden Abschnitt (7) der Längsbohrung (5), sich in diesem Abschnitt (7) befindliche und parallel zur Längsachse erstreckende Schlitze (8) in dem Gewindeschaftteil (2), ein sich in der Längsbohrung (5) erstreckendes Innengewinde
und ein Spreizteil (12) aus einem die Spitze (14) der Knochenschraube (1) bildenden Spitzenteil und einem sich in die Längsbohrung (5) erstreckenden Schaft (16) mit einem mit dem Innengewinde zusammenwirkenden ersten Gewindeteil (17).

2. Knochenschraube nach Anspruch 1,
dadurch gekennzeichnet, daß der sich erweiternde Abschnitt (7) im wesentlichen konisch ausgebildet ist und das Spitzenteil (12) einen entsprechenden, sich zum Kopf (3) hin verjüngenden konischen Abschnitt (15) aufweist.

3. Knochenschraube nach einem der Ansprüche 1 oder 2,
dadurch gekennzeichnet, daß das Spitzenteil an seinem dem Schaft (16) zugewandten Ende eine koaxial ausgerichtete Bohrung (13) mit einem Innengewinde und das mit dem Spitzenteil zu verbindende Ende des Schaftes (16) einen damit zusammenwirkenden zweiten Gewindeteil (18) aufweist und daß die Steigrichtung von erstem und zweitem Gewindeteil (17,18) entgegengerichtet sind.

4. Knochenschraube nach einem der Ansprüche 1 bis 3,
dadurch gekennzeichnet, daß der Durchmesser des das Innengewinde aufweisenden Abschnittes (11) der Längsbohrung (5) größer ist als der sich daran anschließende und sich zum freien Ende (6) hin erstreckende Abschnitt der Längsbohrung (5).

5. Knochenschraube nach einem der Ansprüche 1 bis 4,
dadurch gekennzeichnet, daß der Kopf (3) einen relativ zum Gewindeschaftteil beweglichen Aufnahmeteil (20) aufweist.

## Claims

1. Bone screw (1) with a threaded shank part (2) and a head (3), characterized by a longitudinal bore (5) extending along the longitudinal axis of the threaded shank part (2), a section (7) widening towards the free end (6) of the longitudinal bore (5) remote from the head, slots (8) situated in this section (7) of the threaded shank part (2) and extending parallel to the longitudinal axis, an internal thread extending in the longitudinal bore (5), and a spreading part (12) consisting of a point part forming the point (14) of the bone screw (1) and a shank (16) which extends into the longitudinal bore (5) and which has a first thread part (17) cooperating with the internal thread.

2. Bone screw according to Claim 1, characterized in that the widening section (7) is designed essentially conically, and the spreading part (12) has a corresponding conical section (15) tapering towards the head (3).

3. Bone screw according to one of Claims 1 and 2, characterized in that the point part has at its end facing the shank (16) a coaxially aligned bore (13) with an internal thread, and that end of the shank (16) to be connected to the point part has a second thread part (18) cooperating therewith, and in that the pitches of first and second thread parts (17, 18) are in opposite directions.

4. Bone screw according to one of Claims 1 to 3, characterized in that the diameter of that section (11) of the longitudinal bore (5) having the internal thread is greater than the adjacent section of the longitudinal bore (5) extending towards the free end (6).

5. Bone screw according to one of Claims 1 to 4, characterized in that the head (3) has a receiving part (20) which is movable relative to the threaded shank part.

## Revendications

1. Vis pour os (1) avec une partie de tige filetée (2) et une tête (3),
caractérisée par un passage longitudinal (5), qui s'étend le long de l'axe longitudinal de la partie de tige filetée (2),
par une partie (7) du passage longitudinal (5) qui va en s'élargissant en direction de l'extrémité (6) située à l'opposé de la tête, par une fente (8) située dans cette partie (7) et s'étendant parallèlement à l'axe longitudinal, dans la partie de tige filetée (2), un filet intérieur s'étendant dans le passage longitudinal (5),
et par une pièce extensible (12) consistant en une partie de pointe, constituant la pointe (14) de la vis pour os (1), et en une tige (16), qui s'étend dans le passage longitudinal (5) et qui comporte une première partie filetée (17) qui coopère avec le filet intérieur.

2. Vis pour os selon la revendication 1,
caractérisée en ce que la partie (7), qui va en s'élargissant est de forme sensiblement conique et en ce que la pièce formant pointe (12) présente une partie conique (15) correspondante qui va en s'amincissant en direction de la tête (3).

3. Vis pour os selon l'une des revendications 1 ou 2,
caractérisée en ce que la pièce formant pointe présente, à son extrémité tournée vers la tige (16), un passage (13) orienté de manière coaxiale avec un filet intérieur, en ce que l'extrémité de la tige (16) à relier à la pièce formant pointe présente une deuxième partie filetée (18) pour coopérer avec elle, et en ce que les première et deuxième parties filetées (17, 18) ont des pas en sens contraires.

4. Vis pour os selon l'une des revendications 1 à 3,
caractérisée en ce que le diamètre de la partie (11) du passage longitudinal (5) présentant le filet intérieur est supérieur à celui de la partie du passage longitudinal (5), qui y fait suite et qui s'étend jusqu'à l'extrémité libre (6).

5. Vis pour os selon l'une des revendications 1 à 4,
caractérisée en ce que la tête (3) présente un élément de prise (20) mobile par rapport à la partie de tige filetée.
